# EUROPEAN PATENT APPLICATION

(11) **EP 1 388 530 A1**
(43) Date of publication of application: **11.02.2004**
(21) Application number: 02724761.8
(22) Date of filing: 10.05.2002
(51) Int. Cl.: C07C 29/145, C07C 29/78, C07C 31/26

(54) **METHOD FOR PRODUCING SUGAR ALCOHOL**

(30) Priority: 11.05.2001 JP 2001141287
(71) Applicant: Fushimi Pharmaceutical Company, Limited, Marugame-shi, Kagawa 763-8605 (JP)
(72) Inventor: YOSHINO, A. Fushimi Pharmaceutical Company, Ltd., Marugame-shi, Kagawa 763-8605 (JP); IZUMORI, Ken, Takamatsu-shi, Kagawa 761-8078 (JP); TAKAHASHI, T. Fushimi Pharmaceutical Company, Ltd., Marugame-shi, Kagawa 763-8605 (JP)
(74) Representative: VOSSIUS & PARTNER
(86) International application number: PCT/JP2002/004564
(87) International publication number: WO 2002/092545

(57) **Abstract**

A process for producing sugar alcohols having six carbon atoms, which comprises hydrogenating ketohexose, such as psicose, tagatose, sorbose and the like, in the presence of a catalyst containing a metal selected from the elements belonging to the eighth family in the periodic table, such as nickel, ruthenium, platinum, palladium and the like, is provided. According to this process, sugar alcohols having six carbon atoms can be produced efficiently at a large amount, the separation and recovering of the catalyst after completing the reaction are facilitated, and sugar alcohols having a desired production ratio can be produced efficiently.

## Description

### TECHNICAL FIELD

The present invention relates to processes for producing sugar alcohols having 6 carbon atoms (hexitol). More specifically, the present invention relates to processes for producing sugar alcohols having 6 carbon atoms such as talitol, alitol, iditol and the like through a hydrogenation reaction from ketohexose in the presence of a specific catalyst(s).

### BACKGROUND ART

Sugar alcohols such as sorbitol, mannitol and the like have been used in a variety of uses. Although talitol, alitol, iditol and the like are sugar alcohols that scarcely exist in nature, they are expected to be used for foods, cosmetics, pharmaceutical products, chemical products, pesticides, plant growth regulators and the like. D-talitol and alitol are respectively represented by the following formulae [1] and [2].

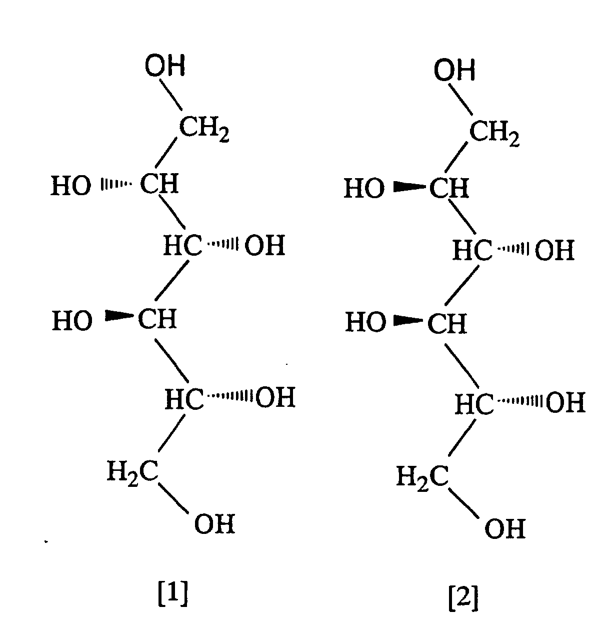

As examples of the processes for producing these sugar alcohols, isolation and identification of D-talitol from *Himanthalia elongate,* isolation and identification of alitol from *Iteailicifolia,* and the like are reported heretofore. However, it is very difficult to yield D-talitol and alitol at a large amount according to such processes.

In addition, also known involve the fermentation and production of D-talitol from D-psicose, a ketohexose represented by the formula [3] below, by *Candida famata*, the fermentation and production of D-talitol from D-tagatose by *Aureobasidium Pullulans*, the fermentation and production of alitol from D-psicose by *Enterobacter agglomerans* (Journal of Fermentation and Bioengineering, 79, 323-327, 1995). However, quantitative productivity is inferior in such fermentation and production by the microorganisms. Thus, these techniques were far from being assessed as valuable processes for producing D-talitol and alitol at a large amount.

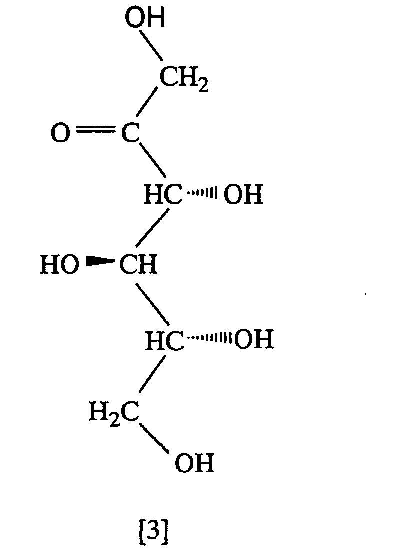

Exemplary processes for producing at a large amount may also include a process in which D-psicose is reduced (hydrogenated) using sodium borohydride. However, this process involves a problem that separation and recovery of sodium borohydride from the product is difficult after completing the reaction.

Production processes utilizing microorganisms for producing sugar alcohols having six carbon atoms other than D-talitol and alitol, such as L-talitol and iditol, are also known. However, according to the processes, it is difficult to obtain the sugar alcohols at a large amount, likewise producing D-talitol and alitol.

An aspect of the present invention is to solve the problems in the prior art as described above, and to provide a process for the efficient production of sugar alcohols having six carbon atoms at a large amount by a hydrogenation reaction of a ketohexose with enabling the facilitated separation and recovering of the catalyst after completing the reaction.

By the prior art, it was difficult to selectively obtain intended sugar alcohols from a mixture of stereoisomers such as D- or L-talitol, alitol, galactitol and the like.

Another aspect of the present invention is to provide a process for readily obtaining a mixture of two or more kinds of intended sugar alcohols having extremely low content of the impurities other than the intended sugar alcohols, and having a desired ratio among the two or more kinds of sugar alcohols.

As a consequence of elaborate investigations for a process for producing sugar alcohols such as D-talitol and alitol from a ketohexose such as D-psicose by a hydrogenation reaction, the present inventors found that the above problems could be solved by conducting a hydrogenation reaction in the presence of a specific metal catalyst. Thus, the present invention was completed.

### DISCLOSURE OF INVENTION

Accordingly, the present invention provides a process for producing sugar alcohols having six carbon atoms, which comprises hydrogenating a ketohexose in the presence of a catalyst containing a metal selected from the elements belonging to the eighth family in the periodic table.

### BEST MODE FOR CARRING OUT THE INVENTION

Examples of the ketohexose used as the raw material in the process of the present invention for producing sugar alcohols include D-psicose, L-psicose, D-tagatose, L-tagatose, D-sorbose, L-sorbose and L-fructose. A mixture of two or more selected from them can also be used.

Ketohexose such as psicose, tagatose and the like was a rare sugar in the past, which was difficult to obtain at a large amount. However, production of the ketohexose having high purity at a large amount was enabled by a reaction using an epimerase at present. For example, an efficient production of D-psicose from D-fructose using D-ketohexose 3-epimerase was enabled, and thus the production at a large amount was allowed at approximately 100% purity on high performance liquid chromatography (HPLC) (Journal of Fermentation and Bioengineering, 80, 101-103, 1995).

In the production process of the present invention, a ketohexose that was produced in such a manner can be used as a raw material.

The sugar alcohol produced according to the production process of the present invention has six carbon atoms.

D-talitol and alitol can be produced from D-psicose. L-talitol and alitol can be produced from L-psicose. D-talitol and galactitol can be produced from D-tagatose. L-talitol and galactitol can be produced from L-tagatose. D-iditol and L-sorbitol can be produced from D-sorbose. L-iditol and D-sorbitol can be produced from L-sorbose.

The metal catalyst used in the process for producing sugar alcohols according to the present invention is a catalyst containing a metal selected from the elements belonging to the eighth family in the periodic table.

The elements belonging to the eighth family of the periodic table refer to the elements belonging to the iron, cobalt, nickel and platinum family. The elements belonging to the platinum family refer to six elements: ruthenium, rhodium, palladium, osmium, iridium and platinum. Among the metals selected from the elements belonging to the eighth family in the periodic table, metals selected from the elements belonging to the nickel and platinum family are preferably used as a catalyst in the present invention. A metal selected from nickel, ruthenium, platinum and palladium is more preferable.

Among these, in respect to the hydroxylation ability, ruthenium and platinum are preferable. In the process for producing D-talitol and alitol from D-psicose, ruthenium and platinum exhibit higher hydroxylation ability than palladium. In addition, the hydrogenation can be effected under a condition of low temperature and low pressure. They were verified to have more potent catalytic ability than that of copper chromium, Raney cobalt and the like. For confirmation, the present inventors used powder of copper chromium catalyst N203 (manufactured by Nikki Chemical Co., Ltd), and found that catalytic activity of this catalyst was scarcely verified.

In addition, characteristics of the catalyst used in the reductive reaction may not be determined by the kind of the metal alone, but affected by a carrier on which the metal is supported.

Examples of the carrier used in the present invention onto which the metal catalyst is supported include activated charcoal, metal oxides such as titanium oxide, alumina and the like, barium sulfate, diatomaceous earth, and the like.

Further, so called Raney nickel catalyst that is obtained by the treatment of an alloy of nickel and aluminum or the like with caustic alkali is preferable because it can accelerate the reaction velocity by the increase in the surface area, that is, because of high catalytic activity of the same.

Mode for carrying out the process for producing sugar alcohols according to the present invention is not particularly limited. However, in general, a ketohexose is dissolved in a solvent such as water and the like, and thereafter a metal catalyst as described above is added thereto. Thus resultant mixture is placed into a pressure vessel, and then a hydrogenation reaction of the ketohexose is effected by pressing hydrogen into the vessel.

In this case, the solvent used is generally water. However, alcohol solvent such as ethanol and the like, methyl acetate, ethyl acetate, and the mixture thereof as well as the mixed solvent of water and the same may also be used.

The concentration of ketohexose in the reaction mixture is usually from 1 to 60 w/v%, preferably from 5 to 50 w/v%.

Moreover, the reaction temperature is usually from 10 to 150°C, preferably from 10 to 70°C, and more preferably from 30 to 60°C in order to prevent the occurrence of byproducts of the reaction.

The reaction pressure is usually in a condition of from 1 to 200 kg/cm² (gauge pressure), preferably of from 5 to 100 kg/cm².

The progress of the reaction can be confirmed by the analysis of the ketohexose and the yielded sugar alcohols in samples taken from the reaction mixture at regular time intervals. For the analysis of ketohexose and yielded sugar alcohols, HPLC or the like is preferably used.

After completing the reaction, the catalyst can be readily removed by filtration, decantation, centrifugal separation, or the like. Through removing the catalyst in such a manner, a solution containing the intended sugar alcohols can be obtained.

In the process for producing sugar alcohols according to the present invention, the production ratio of yielded sugar alcohols, for example the production ratio of D-talitol and alitol, can be changed by adding an optically active substance for inducing the diastereo isomer, that is, an asymmetric source. Examples of such an asymmetric source include boric acid, cinchonidine, cinchonine, ephedrine, quinidine, brucine and the like; alkaloids such as quinine, strychnine and the like; sugar and derivatives thereof such as D-mannitol and the like; menthol, camphor, terpene, hydroxyl acids such as L-tartaric acid, L-lactic acid, L-malic acid and the like; and amino acids such as L-leucine, L-cystine, L-cysteine and the like. In addition, the production ratio of sugar alcohols can be also changed by using a synthetic asymmetric source, which was molecular designed aiming at diastereo isomeric reduction, together with a catalyst containing a metal selected from nickel, ruthenium, platinum and palladium. Examples of such a synthetic asymmetric source include asymmetric phosphine such as existing BINAP and the like.

Moreover, in the process for producing sugar alcohols according to the present invention, the ratio of production of the yielded sugar alcohols, D-talitol and alitol, for example, can be also changed depending on the kinds of the metal catalyst that is used. More specifically, the production ratio of two or more kinds of sugar alcohols varies depending on the conditions such as kinds of the metal of the catalyst, the kinds of the carrier, and the like.

In the production of D-talitol and alitol, Raney nickel is preferably used for giving the product of D-talitol : alitol in the range of from approximately 50 : 50 to 40 : 60, whilst platinum is preferably used for giving the product of D-talitol : alitol in the range of from approximately 30 : 70 to 42 : 58, in general.

In the production of D-talitol and galactitol from D-tagatose, ruthenium is preferably used for giving the product of D-talitol : galactitol in the range of from approximately 30 : 70 to 40 : 60, whilst platinum is preferably used for giving the product of D-talitol : galactitol in the range of from approximately 60 : 40 to 80 : 20, in general.

In the production of L-talitol and galactitol from L-tagatose, ruthenium is preferably used for giving the product of L-talitol : galactitol in the range of from approximately 30 : 70 to 40 : 60.

In the production of D-iditol and L-sorbitol from D-sorbose, ruthenium as well as platinum are preferably used for giving the product of D-iditol : L-sorbitol in the range of from approximately 70 : 30 to 50 : 50.

In the production of D-sorbitol and L-iditol from L-sorbose, ruthenium as well as platinum are preferably used for giving the product of D-sorbitol : L-iditol in the range of from approximately 40 : 60 to 50 : 50.

Accordingly, a mixture containing a specific sugar alcohol such as alitol, galactitol and the like at a large amount can be readily obtained, and thus it becomes easy to obtain the pure specific sugar alcohol.

The present inventors found that the manipulation of crystallization, in which crystals are precipitated from an aqueous solution of a mixture of D-talitol and alitol through concentration or the like, results in a mixture containing the greater ratio of alitol than the ratio for the pre-crystallization material. By repeating this manipulation of crystallization, or by washing the crystal thus crystallized containing the greater ratio of alitol with a solution of alitol, pure alitol can be obtained. According to a similar manner, crystal containing the greater ratio of alitol can be obtained from an aqueous solution of a mixture of L-talitol and alitol. In addition, the manipulation of crystallization, in which crystals are precipitated from an aqueous solution of a mixture of galactitol and D- or L-talitol, through concentration or the like, results in a mixture containing the greater ratio of galactitol than the ratio for the pre-crystallization material, and by repeating this manipulation of crystallization, pure galactitol can be obtained.

In this case, when a mixture containing alitol or galactitol at a large amount, which was obtained by the process according to the present invention, is used as a raw mixture, frequency of the manipulation of the crystallization can be decreased. In addition, advantages in respect to the yield and to the facilitation of other manipulation can be achieved, for example.

D-talitol, alitol, L-talitol and galactitol can be respectively purified and separated by chromatography from a mixture of D- or L-talitol and alitol, or a mixture of galactitol and D- or L-talitol. However, to obtain pure alitol or galactitol is enabled in an easier manner by the present process and the manipulation of crystallization.

Further, after pure alitol or a mixture containing greater ratio of alitol is obtained according to the above mentioned process, the remainder, that is a mixed solution of alitol and D-talitol, can be subjected to a separation by chromatography to obtain pure D-talitol efficiently. Similarly, after pure galactitol or a mixture containing greater ratio of galactitol is obtained, the remainder, that is a mixed solution of D-talitol or L-talitol and galactitol, can be subjected to a separation by chromatography to obtain pure D-talitol or L-talitol efficiently. That is, by separating pure alitol or galactitol, or a mixture containing greater ratio thereof, from the mixed solution, the content of D-talitol or L-talitol becomes higher and the content of alitol or galactitol becomes lower than those in the mixed solution before the separation.

Alitol or galactitol and D-talitol or L-talitol can be separated by chromatography from a solution of mixture of Alitol or galactitol and D-talitol or L-talitol. When the content of alitol or galactitol in the mixture is large, amount of the eluted fraction containing only D-talitol or L-talitol is small. Therefore, using remainder of the mixed solution after separation of pure alitol or galactitol, or a mixture containing greater ratio thereof, the amount of eluted fraction containing pure D-talitol or L-talitol becomes larger, and by using the fraction, pure D-talitol or L-talitol can be produced efficiently.

That is, the present invention also provides a process for producing D-talitol, which comprises a manipulation wherein alitol is precipitated from a mixed aqueous solution of D- or L-talitol and alitol, and the remainder of the mixed aqueous solution after the precipitation is passed through a chromatography column to obtain D- or L-talitol fraction not containing alitol; and a process for producing D- or L-talitol, which comprises a manipulation wherein galactitol is precipitated from a mixed aqueous solution of galactitol and D- or L-talitol, and the remainder of the mixed aqueous solution after the precipitation is passed through a chromatography column to obtain D- or L-talitol fraction not containing galactitol.

The same conditions for chromatography can be adopted as the conditions adopted in usual separations.

The present invention is described concretely by the examples below. However, the present invention is not limited to these examples.

### Example 1

### (1) Activation of Raney nickel

To 10 g of 50% Raney nickel (manufactured by Wako Pure Chemicals Inc.), was added 100 g of 20% aqueous solution of NaOH. Following the addition, the mixture was heated at 90° for 1 hour. After confirming the termination of the generation of bubble, the catalyst was washed with distilled water by decantation. The washes were carried out until pH of the lavage becomes 9.2.

### (2) Production of D-talitol and alitol

To a 1L glass autoclave equipped with a stirrer and a thermometer, was added 300 g of an aqueous solution containing 100 g of D-psicose, to which 24 g of Raney nickel obtained by the procedure as described above was added. Water was further added to adjust the total volume of the reaction mixture being 600 g. Then, calcium carbonate was added thereto to adjust the pH of the reaction mixture being 7. The reaction was conducted keeping the conditions of at the temperature of 50°C, at the hydrogen pressure of 12 kg/cm² (gauge pressure), and at the rate of stirring of 700 rpm. At regular time intervals, a sample was taken from the reaction mixture, which was subjected to the analysis of D-psicose, D-talitol and alitol to confirm the progress status of the reaction. For performing the analysis of D-psicose, D-talitol and alitol, HPLC was employed. The analytical conditions were: using a column of Hitachi HPLC packed column Gelpack GL-C611; at a column temperature of 60°C; at a flow rate of 1.0 ml/min; and detecting using an RI detector. The sample subjected to the analysis was prepared by appropriately diluting the sample from the reaction mixture taken with time, followed by desalting through the addition of an anion exchange resin and a cation exchange resin. As a result, D-psicose decreased to 1% in the reaction of 8 hours, and the resulting production ratio of D-talitol and alitol was 55 : 45. Thereafter, the reaction mixture was filtrated through a 5C filter (manufactured by ADVANTEC, Inc.). Upon the analysis of thus resultant filtrate, it was revealed that the filtrate contained 53 g of D-talitol and 42 g of alitol, which exhibited 98% of the purity of sugar alcohols that are the combination of the two sugars, D-talitol and alitol, in the liquid chromatography analysis.

### Example 2

To a 1L glass autoclave equipped with a stirrer and a thermometer, was added an aqueous solution containing 50 g of D-psicose, to which 30 g of platinum activated charcoal powder N1093C3 (manufactured by Nikki Chemical Co., Ltd.) was added. Water was thereafter added to adjust the total volume of the reaction mixture being 250 g. Then, calcium carbonate was added thereto to adjust the pH of the reaction mixture being 7. The reaction was conducted keeping the conditions of at the temperature of 40°C, at the hydrogen pressure of 12 kg/cm² (gauge pressure), and at the rate of stirring of 600 rpm. The analysis of D-psicose, D-talitol and alitol was performed using HPLC under similar conditions to those in Example 1. As a result, D-psicose decreased to 13% in the reaction of 17 hours, and to 0.3% in the reaction of 68 hours. The resulting production ratio of D-talitol and alitol was 35 : 65. Thereafter, the reaction mixture was filtrated through a 5C filter (manufactured by ADVANTEC, Inc.). Upon the analysis of thus resultant filtrate, it was revealed that the filtrate contained 16 g of D-talitol and 32 g of alitol, which exhibited 99% of the purity of sugar alcohols that are the combination of the two sugars, D-talitol and alitol, in the liquid chromatography analysis.

### Example 3

To a 1L glass autoclave equipped with a stirrer and a thermometer, was added an aqueous solution containing 10 g of D-psicose, to which 2 g of 5% ruthenium carbon powder (manufactured by N E Chemcat Corporation) was added. Water was thereafter added to adjust the total volume of the reaction mixture being 200 g. Then, calcium carbonate was added thereto to adjust the pH of the reaction mixture being 7. The reaction was conducted keeping the conditions of at the temperature of 40°C, at the hydrogen pressure of 12 kg/cm² (gauge pressure), and at the rate of stirring of 400 rpm. The analysis of D-psicose, D-talitol and alitol was performed using HPLC under similar conditions to those in Example 1. As a result, D-psicose decreased to 63% in the reaction of 7.5 hours. The resulting production ratio of D-talitol and alitol was 62 : 38. Thereafter, the reaction mixture was filtrated through a 5C filter (manufactured by ADVANTEC, Inc.). Upon the analysis of thus resultant filtrate, it was revealed that the filtrate contained 2.2 g of D-talitol and 1.1 g of alitol.

### Example 4

To a 1L glass autoclave equipped with a stirrer and a thermometer, was added an aqueous solution containing 10 g of D-psicose, to which 30 g of platinum activated charcoal powder N1093C3 (manufactured by Nikki Chemical Co., Ltd.) and 0.05 g of cinchonidine were added. Water was thereafter added to adjust the total volume of the reaction mixture being 200 g. Then, calcium carbonate was added thereto to adjust the pH of the reaction mixture being 7. The reaction was conducted keeping the conditions of at the temperature of 40°C, at the hydrogen pressure of 12 kg/cm² (gauge pressure), and at the rate of stirring of 600 rpm. The analysis of D-psicose, D-talitol and alitol was performed using HPLC under similar conditions to those in Example 1. As a result, D-psicose decreased to 17% in the reaction of 17 hours, and to 1.7% in the reaction of 44 hours. The resulting production ratio of D-talitol and alitol was 31 : 69. Thereafter, the reaction mixture was filtrated through a 5C filter (manufactured by ADVANTEC, Inc.). Upon the analysis of thus resultant filtrate, it was revealed that the filtrate contained 3 g of D-talitol and 6.7 g of alitol, which exhibited 99% of the purity of sugar alcohols that are the combination of the two sugars, D-talitol and alitol, in the liquid chromatography analysis.

### Example 5

Concentration and crystallization of 2050 g of a mixed solution of alitol and D-talitol was carried out, which was obtained by 100% reduction of 270 g of D-psicose by the hydrogenation using Raney nickel as a catalyst. The mixture was subjected to vacuum suction filtration for the separation from the mother liquid to recover the crystal. The resultant crystal is referred to as primary crystal. The primary crystal was recrystallized, followed by repeating the similar processes to give secondary crystal and tertiary crystal. Forty-eight g of alitol was obtained by the manipulation of crystallization repeated three times. Similar analysis to that in Example 1 revealed that the resulting tertiary crystal had 100% purity of alitol. On the other hand, 217 g of mixed sugar alcohols of alitol and D-talitol were obtained from the mixed solution of the mother liquids resulting from the separation of the crystal in the manipulation described above.

HPLC analysis was conducted to obtain the ratio of alitol and D-talitol in the crystallization water as well as in the mother liquid, and the results thereof are shown in Table 1.

**Table 1**

| | Yield of sugars | Alitol | D-talitol |
|---|---|---|---|
| Mixed aqueous solution of alitol and D-talitol | 271 g | 48% | 52% |
| Primary crystal | 56 g | 91% | 9% |
| Secondary crystal | 50 g | 97% | 3% |
| Tertiary crystal | 48 g | 100% | 0% |
| Mixed mother liquid | 217 g | 37% | 63% |

As is clear from the results in the Table above, alitol can be crystallized in preference, and pure alitol can be obtained through the repeated manipulation of crystallization from the mixed aqueous solution of alitol and D-talitol. The mixed aqueous solution of alitol and D-talitol, which was obtained by the process according to the present invention using a platinum catalyst and the like, has greater content of alitol, thereby enabling pure alitol to yield in an easier manner. Further, by the analogy of crystallization experiment of galactitol shown in Example 11 below, alitol in high purity can be obtained by washing the primary crystal obtained from a mixed aqueous solution of alitol and D-talitol with a solution of alitol.

### Example 6

To a 1L glass autoclave equipped with a stirrer and a thermometer (TES-1 type, manufactured by TAIATSU TECHNO CORPORATION), was added an aqueous solution containing 1.35 g of L-sorbose, to which 13.0 g of platinum activated charcoal powder N1093B3 (manufactured by Nikki Chemical Co., Ltd.) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of L-sorbose, L-iditol and D-sorbitol was performed with HPLC according to the similar conditions to those in Example 1. The sample subjected to the analysis was prepared by appropriately diluting the reaction mixture, followed by desalting through the addition of a CO₃²⁻ type anion exchange resin, Amberlite IRA411S, and H⁺ type Diaion SK1B.

The results revealed that the reaction ratio after the reaction of 2 hours was 4.0 %. After completing the reaction, the reaction mixture was filtrated through a 5C filter (manufactured by ADVANTEC, Inc.) to remove the catalyst. Upon the HPLC analysis, the production ratio of L-iditol and D-sorbitol was 55 : 45.

### Example 7

To a 1L glass autoclave equipped with a stirrer and a thermometer (TES-1 type, manufactured by TAIATSU TECHNO CORPORATION), was added an aqueous solution containing 1.35 g of L-sorbose, to which 3.0 g of 5% ruthenium carbon powder (manufactured by N E Chemcat Corporation) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of L-sorbose, L-iditol and D-sorbitol was performed with HPLC according to the similar conditions to those in Example 1. The sample subjected to the analysis was prepared by appropriately diluting the reaction mixture, followed by desalting through the addition of a CO₃²⁻ type anion exchange resin, Amberlite IRA411S, and H⁺ type Diaion SK1B.

The results revealed that the reaction ratio after the reaction of 2 hours was 100 %. After completing the reaction, the reaction mixture was filtrated through a 5C filter (manufactured by ADVANTEC, Inc.) to remove the catalyst. Upon the HPLC analysis, the production ratio of L-iditol and D-sorbitol was 58 : 42.

### Example 8 (Separation and purification of D-talitol)

A mixed solution (concentration: 30%) of alitol : D-talitol in the ratio of 11 : 89, that was obtained by removing a part of alitol from a mixture of alitol : D-talitol according to the similar manner to that in Example 5, was passed through a liquid chromatography column. The passed volume was 5 **%** of the column volume. Considering occurrence of breeding of microorganisms and precipitation of sugar alcohol, the column temperature was adjusted at 60 °C and the flow rate was adjusted at the space volume of SV = 0.5 (h⁻¹). The fraction of elution of the first 2.7 L, in which alitol were not separated from talitol completely, was removed, and the following fraction containing pure D-talitol was obtained. By concentrating the fraction of pure D-talitol, crystal of D-talitol having purity of 99 % or more was obtained.

### Example 9

To a 1L glass autoclave equipped with a stirrer and a thermometer (TES-1 type, manufactured by TAIATSU TECHNO CORPORATION), was added an aqueous solution containing 0.18 g of D-tagatose, to which 17.3 g of platinum activated charcoal powder N1093B3 (manufactured by Nikki Chemical Co., Ltd.) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of D-tagatose, D-talitol and galactitol was performed with HPLC according to the similar conditions to those in Example 1.

The results revealed that the reaction ratio after the reaction of 2 hours was 5.0 %. After completing the reaction, the reaction mixture was filtrated through a 5C filter (manufactured by ADVANTEC, Inc.) to remove the catalyst. Upon the HPLC analysis, the production ratio of D-talitol and galactitol was 67 : 33.

### Example 10

To a 1L glass autoclave equipped with a stirrer and a thermometer (TES-1 type, manufactured by TAIATSU TECHNO CORPORATION), was added an aqueous solution containing 0.18 g of D-tagatose, to which 0.4 g of 5% ruthenium carbon powder (manufactured by N E Chemcat Corporation) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of D-tagatose, D-talitol and galactitol was performed with HPLC according to the similar conditions to those in Example 1.

The results revealed that the reaction ratio after the reactions of 2 hour was 100 %. After completing the reaction, the reaction mixture was filtrated through a 5C filter (manufactured by ADVANTEC, Inc.) to remove the catalyst. Upon the HPLC analysis, the production ratio of D-talitol and galactitol was 34 : 66.

### Example 11(Crystallization and purification of galactitol)

A solution obtained by dissolving 3.4 g of D-talitol and 6.6 g of galactitol in 250 ml of distilled water was concentrated to 40 g at 60 °C under reduced pressure. After leaving as it is for 3 hours at 5 °C, the resultant was filtrated through a 5C filter (manufactured by ADVANTEC, Inc.)to recover the filtrate 1. The mixed crystal on the filter was washed with 15 ml of 5 % galactitol solution. The wash was conducted two times, and respectively, lavage 2 and lavage 3, and 4.5 g of crystal 4 were obtained. Purity on HPLC of the resultant crystal was 99.2 %. Results of the HPLC analysis were shown in Table 2.

**Table 2**

| | D-talitol | galactitol |
|---|---|---|
| Filtrate 1 | 2.7 g | 1.6 g |
| lavage 2 | 0.6 g | 1.1 g (content before wash 0.8 g) |
| lavage 3 | 0.1 g | 0.9 g (content before wash 0.8 g) |
| Crystal 4 | 0.04 g | 4.4 g |

As is clear from the results in the Table above, galactitol having high purity can be obtained by washing the crystal crystallized from a mixed aqueous solution of galactitol and D-talitol with a solution of galactitol. Using a mixed solution obtained according to the above process and containing high content of galactitol, pure galactitol can be obtained in an easier manner.

It is expected that galactitol can be crystallized in preference from a mixed solution of L-talitol and galactitol according to the same manner as above.

### Example 12

To a 1L glass autoclave equipped with a stirrer and a thermometer (TES-1 type, manufactured by TAIATSU TECHNO CORPORATION), was added an aqueous solution containing 0.18 g of L-tagatose, to which 0.41 g of 5% ruthenium carbon powder (manufactured by N E Chemcat Corporation) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of L-tagatose, L-talitol and galactitol was performed with HPLC according to the similar conditions to those in Example 1.

The results revealed that the reaction ratio after the reaction of 2 hours was 100 %. After completing the reaction, the reaction mixture was filtrated through a 5C filter (manufactured by ADVANTEC, Inc.) to remove the catalyst. Upon the HPLC analysis, the production ratio of L-talitol and galactitol was 32 : 68.

### Example 13

To a 1L glass autoclave equipped with a stirrer and a thermometer (TES-1 type, manufactured by TAIATSU TECHNO CORPORATION), was added an aqueous solution containing 0.18 g of D-sorbose, to which 17.3 g of platinum activated charcoal powder N1093B3 (manufactured by Nikki Chemical Co., Ltd.) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of D-sorbose, L-sorbitol and D-iditol was performed with HPLC according to the similar conditions to those in Example 1.

The results revealed that the reaction ratio after the reaction of 2 hours was 2.5 %. After completing the reaction, the reaction mixture was filtrated through a 5C filter (manufactured by ADVANTEC, Inc.) to remove the catalyst. Upon the HPLC analysis, the production ratio of L-sorbitol and D-iditol was 38 : 62.

### Example 14

To a 1L glass autoclave equipped with a stirrer and a thermometer (TES-1 type, manufactured by TAIATSU TECHNO CORPORATION), was added an aqueous solution containing 0.18 g of D-sorbose, to which 0.4 g of 5% ruthenium carbon powder (manufactured by N E Chemcat Corporation) was added. Water was further added to adjust the total volume of the reaction mixture being 150 g. Then, the pH of the reaction mixture was adjusted to 7.

Thereafter, hydrogen gas was blown into the reaction vessel, and the reaction was conducted keeping the conditions of at the temperature of 40°C, at the hydrogen pressure of 12 kg/cm² (gauge pressure), and at the rate of stirring of 500 rpm. Before starting the reaction, the atmosphere in the vessel was replaced with hydrogen gas.

The analysis of D-sorbose, L-sorbitol and D-iditol was performed with HPLC according to the similar conditions to those in Example 1.

The results revealed that the reaction ratio after the reaction of 2 hours was 100 %. After completing the reaction, the reaction mixture was filtrated through a 5C filter (manufactured by ADVANTEC, Inc.) to remove the catalyst. Upon the HPLC analysis, the production ratio of L-sorbitol and D-iditol was 36 : 64.

In accordance with the process for producing sugar alcohols according to the present invention, intended sugar alcohols can be efficiently produced from ketohexose at a large amount.

Further, the catalyst can be readily separated and recovered by filtrating from the solution containing the sugar alcohols, unlike the case where the reduction by sodium borohydride is conducted.

In addition, when a mixture of two or more kinds of sugar alcohols is obtained, the production ratio in the sugar alcohols also can be readily changed by the alteration of kinds of metal catalyst and kinds of asymmetric sources.

The sugar alcohols obtained by the process according to the present invention have extremely low content of impurities, and thus can be used for foods, cosmetics, pharmaceutical products, chemical products, pesticides, plant growth regulators and the like.

Accordingly, the process for producing sugar alcohols according to the present invention has eminent industrial significance in not only sugar-manufacturing industry, but also in related industries such as foods, cosmetics, pharmaceutical industries.

## Claims

1. A process for producing sugar alcohols having six carbon atoms, which comprises hydrogenating ketohexose in the presence of a catalyst containing a metal selected from the elements belonging to the eighth family in the periodic table.

2. The process for producing sugar alcohols according to claim 1 wherein the elements belonging to the eighth family in the periodic table are selected from the elements belonging to the nickel and platinum family.

3. The process for producing sugar alcohols according to claim 2 wherein the elements belonging to the nickel and platinum family are selected from nickel, ruthenium, platinum and palladium.

4. The process for producing sugar alcohols according to claim 2 wherein the elements belonging to the nickel and platinum family are selected from ruthenium and platinum.

5. The process for producing sugar alcohols according to claim 1 wherein the catalyst containing a metal is Raney nickel.

6. The process for producing sugar alcohols according to any one of claims 1 to 5 wherein the reaction of hydrogenation is conducted in an aqueous solution of ketohexose.

7. The process for producing sugar alcohols according to claim 6 wherein the reaction temperature is from 10 to 150°C, and the reaction pressure is from 1 to 200 kg/cm².

8. The process for producing sugar alcohols according to any one of claims 1 to 7 wherein the hydrogenation is conducted in the presence of an asymmetric source.

9. The process for producing sugar alcohols according to any one of claims 1 to 8 wherein the ketohexose is at least one selected from D-psicose, L-psicose, D-tagatose, L-tagatose, D-sorbose, L-sorbose and L-fructose.

10. The process for producing sugar alcohols according to any one of claims 1 to 9 wherein the ketohexose is D-psicose, and the sugar alcohols are D-talitol and alitol.

11. A process for producing alitol, which comprises a manipulation precipitating alitol from a mixed aqueous solution of alitol and D- or L-talitol.

12. A process for producing galactitol, which comprises a manipulation precipitating galactitol from a mixed aqueous solution of galactitol and D- or L-talitol.

13. A process for producing D- or L-talitol, which comprises a manipulation wherein a mixed aqueous solution is obtained by precipitating and removing alitol from a mixed aqueous solution of alitol and D- or L-talitol, followed by passing the remaining mixed aqueous solution through a chromatography column to obtain D- or L-talitol fraction not containing alitol.

14. A process for producing D- or L-talitol, which comprises a manipulation wherein a mixed aqueous solution is obtained by precipitating and removing galactitol from a mixed aqueous solution of galactitol and D- or L-talitol, followed by passing the remaining mixed aqueous solution through a chromatography column to obtain D- or L-talitol fraction not containing galactitol.
